## Europäisches Patentamt

## European Patent Office

## Office européen des brevets

(19)

(11) Veröffentlichungsnummer: **0 159 311**
**B1**

(12) **EUROPÄISCHE PATENTSCHRIFT**

(45) Veröffentlichungstag der Patentschrift:
19.04.89

(21) Anmeldenummer: 85890044.2

(22) Anmeldetag: 18.02.85

(51) Int. Cl.⁴: **A 61 L 2/04 //**
**A61K35/16**

(54) Verfahren zur Inaktivierung von vermehrungsfähigen filtrierbaren Krankheitserregern in Blutprodukten.

(30) Priorität: 09.03.84 AT 792/84
11.10.84 AT 3237/84

(43) Veröffentlichungstag der Anmeldung:
23.10.85 Patentblatt 85/43

(45) Bekanntmachung des Hinweises auf die Patenterteilung:
19.04.89 Patentblatt 89/16

(84) Benannte Vertragsstaaten:
AT BE CH DE FR GB IT LI NL SE

(56) Entgegenhaltungen:
EP-A-0 094 611
WO-A-82/03871

(73) Patentinhaber: **IMMUNO Aktiengesellschaft für chemisch- medizinische Produkte,
Industriestrasse 72, A-1220 Wien (AT)**

(72) Erfinder: **Eibl, Johann, Dr., Gustav Tschermakgasse 2, A-1180 Wien (AT)**
Erfinder: **Schwarz, Otto, Dr., Celtesgasse 5, A-1190 Wien (AT)**
Erfinder: **Elsinger, Fritz, Dr., Eduard Kleingasse 29, A-1130 Wien (AT)**
Erfinder: **Wöber, Günter, Prof. Dr., Carolusstrasse 23, A-2522 Oberwaltersdorf (AT)**
Erfinder: **Philapitsch, Anton, Hans Kudlichgasse 5, A-2490 Ebenfurt (AT)**
Erfinder: **Linnau, Yendra, Dr., Lavendelweg 24, A-1224 Wien (AT)**
Erfinder: **Dorner, Friedrich, Prof. Dr., Peterlinigasse 17, A-1230 Wien (AT)**
Erfinder: **Trambauer, Karl, Ing., Steudelgasse 37-39/1/8, A-1100 Wien (AT)**
Erfinder: **Frechinger, Wolfgang, Nordmanngasse 22, A-1210 Wien (AT)**

(74) Vertreter: **Wolfram, Gustav, Dipl.- Ing., Schwindgasse 7 P.O. Box 205, A-1041 Wien (AT)**

**Beschreibung**

Die Erfindung betrifft ein Verfahren zur Inaktivierung von vermehrungsfähigen filtrierbaren Krankheitserregern, insbesondere Hepatitisviren und unbekannten Erregern, die eventuell AIDS (acquired immune deficiency syndrome) übertragen können, in Blutprodukten unter Anwendung erhöhter Temperatur sowie ein Verfahren zur Herstellung von Blutprodukten unter Anwendung dieses Verfahrens.

Unter Blutprodukten werden Produkte aus menschlichem oder tierischem Blut bzw. Plasma verstanden, die zur therapeutischen, prophylaktischen oder diagnostischen Anwendung bestimmt sind. Solche Produkte können Enzyme, Proenzyme einschließlich Gerinnungsfaktoren, Enzyminhibitoren, Immunglobuline, Albumin, Plasminogen, Fibrinogen, Fibronectin oder Plasma enthalten.

Es ist eine umfangreiche Literatur vorhanden, die sich mit der Hitzeinaktivierung von vermehrungsfähigen filtrierbaren Krankheitserregern in Blutprodukten befaßt.

Die verschiedenen Verfahren umfassen:
- Erhitzen der Blutprodukte in wässeriger Lösung, gegebenenfalls unter Zusatz viruzider Substanzen,
- Erhitzen der Blutprodukte in wässeriger Lösung in Anwesenheit von Stabilisatoren,
- Behandeln der Blutprodukte mit organischen Lösungsmitteln,
- Bestrahlen der Blutprodukte in festem Zustand,
- Erhitzen der Blutprodukte in trockenem Zustand.

Das Bestreben bei allen diesen Inaktivierungsverfahren geht dahin, die potentielle Infektivität der Präparationen aufzuheben, ihre biologische Aktivität aber weitgehend zu erhalten. Dieses Ziel konnte jedoch bisher nur bei Albuminpräparationen erreicht werden, u.zw. durch Erhitzen von wässerigen Albuminlösungen bei einer Temperatur von 60°C während 10 h, da Albumin wesentlich stabiler gegenüber Hitzeeinwirkung ist als alle anderen Blutproteine.

Im einzelnen sind zum Stand der Technik beispielsweise die folgenden Literaturstellen anzuführen:

Die DE-OS-2 916 711 beschreibt ein Verfahren zur Behandlung von Gerinnungsfaktoren enthaltenden Präparationen in wässeriger Lösung unter Anwendung einer Temperatur von 30 bis 100°C, wobei der Lösung der Gerinnungsfaktoren eine Aminosäure und ein Mono-, Oligosaccharid oder Zuckeralkohol zugesetzt werden.

Die EP-A2-0 053 338 beschreibt ein Verfahren zur Inaktivierung von Hepatitis-Viren in Faktor IX und X enthaltenden Präparationen, wobei eine Erwärmung der wässerigen Lösung eines Blutpräparates in Gegenwart von Calciumionen und gegebenenfalls einer Aminosäure und/oder eines Saccharides oder Zuckeralkoholes bei Temperaturen von bis zu 100°C vorgenommen wird.

In der EP-A2-0 035 204 wird ein Verfahren zur Inaktivierung von wässerigen Proteinlösungen, die Faktor VIII, Fibronectin, Globulin, Fibrinogen und andere Proteine enthalten können, beschrieben, wobei die Komposition mit einem Polyol gemischt und die Mischung auf eine Temperatur von 60 bis 75°C erhitzt wird.

In der EP-A2-0 052 827 ist ein Verfahren zur Inaktivierung von Hepatitis-Viren in einer wässerigen, die Faktoren II und VII enthaltenden Lösung in Anwesenheit eines Chelatbildners und gegebenenfalls einer Aminosäure und/oder eines Saccharides oder Zuckeralkoholes beschrieben.

In der US-A-4 379 085 ist ein Verfahren zur Hitzeinaktivierung eines Plasmaproteins, wie $C_1$-Inhibitor oder Faktor IX, in wässeriger Lösung in Gegenwart von Kalium- oder Ammoniumcitrat beschrieben.

In der EP-A2-0 077 870 ist ein Inaktivierungsverfahren beschrieben, bei welchem eine wässerige, Faktor VIII enthaltende Lösung mit Aminosäuren, Monosacchariden, Oligosacchariden, Zuckeralkoholen und Kohlenwasserstoff- oder Hydroxylkohlenwasserstoffcarbonsäuren mit 3 bis 10 Kohlenstoffatomen auf eine Temperatur von 50 bis 80°C erhitzt wird.

In der PCT-Anmeldung WO 83/04371 ist ein Verfahren zum Inaktivieren von Hepatitis-Viren beschrieben, wobei eine das Virus enthaltende Präparation bei einer Temperatur von 4 bis 40°C mit einem Halogenkohlenwasserstoff, insbesondere Chloroform, behandelt wird.

Die EP-B1-0 015 055 beschreibt ein Verfahren zur Behandlung eines Blutproduktes, wobei das Produkt in wasserfreiem Zustand einer Mikrowellenbestrahlung ausgesetzt wird, um vorhandene Mikroorganismen zu inaktivieren.

Rosenberg et al. beschreiben in einer Abhandlung des XII. International Congress on Blood Transfusion, Abstracts, "MIR" Publishers, Moscow 1969, pp. 473 - 475 ein Verfahren zur Inaktivierung von albuminhältigen Präparationen und Fibrinogen in trockenem Zustand durch 10-stündiges Erhitzen auf 60°C.

Die EP-A2-0 094 611 beschreibt ein Verfahren zur Behandlung einer Faktor VIII enthaltenden Komposition im trockenen Zustand mit weniger als 5 Gew.-% (0,05) Wasser, unter Anwendung einer Temperatur von wenigstens 60°C zwecks Inaktivierung von vorhandenen Hepatitis-Viren.

Die veröffentlichte PCT-Anmeldung WO 82/03871 beschreibt ein Verfahren zur Behandlung von Blutgerinnungsenzyme enthaltenden Zubereitungen, wobei diese zur Inaktivierung vorhandener infektiöser Viren im trockenen Zustand erhitzt werden; als trockener Zustand wird ein solcher mit weniger als 5 Gew.-% (0,05) Wasser definiert.

Wie aus der vorhergehenden Übersicht hervorgeht, sind sowohl Verfahren bekannt, bei denen die zu behandelnde, Blutproteine enthaltende Präparation in wässeriger Lösung oder in einem organischen Lösungsmittel suspendiert vorliegt, als auch Verfahren, bei denen die Präparation in trockenem Zustand, z. B. lyophilisiert, der Inaktivierungsbehandlung unterworfen wird. Trotz aller bisherigen Anstrengungen ist es bisher nicht gelungen, eine Inaktivierungsmethode zu entwickeln, die vergleichbar mit der Inaktivierung des Blutproduktes Albumin - gleichermaßen eine Sicherheit gegen Übertragung von Hepatitis-Viren und anderen

2

pathogenen Viren gewährleistet und die Erhaltung der biologischen Aktivität des jeweiligen Blutproduktes sicherstellt. Seit 30 Jahren werden albuminhältige Lösungen, die in Anwesenheit von geeigneten Stabilisatoren 10 h auf 60°C erhitzt worden sind, millionenfach klinisch angewandt, ohne daß dadurch Infektionserkrankungen übertragen worden wären. Wie in Experimenten mit Schimpansen gezeigt werden konnte, werden Hepatitis-Viren, die albuminhältigen Lösungen zugesetzt wurden, durch Erhitzen dieser Lösungen auf 60°C während 10 h vollständig inaktiviert.

Die Erfindung stellt sich die Aufgabe, ein Inaktivierungsverfahren zu schaffen, das bei Erhaltung der biologischen Aktivität des zu behandelnden Blutproduktes eine zuverlässige Sicherheit gegenüber Viren, insbesondere Hepatitis-Viren, erzielt, die der bei der Inaktivierung von albuminhältigen Lösungen erreichbaren Effizienz gleichwertig oder sogar überlegen ist.

Diese Aufgabe wird erfindungsgemäß bei einem Verfahren der eingangs beschriebenen Art dadurch gelöst, daß die Blutprodukte in festem Zustand auf einen Gehalt an Wasser, Methanol oder Äthanol von mehr als 0,05 (5 Gew.-%) und weniger als 0,70 (70 Gew.-%), vorzugsweise weniger als 0,40 (40 Gew.-%), eingestellt und in einem geschlossenen Behälter bei einer Temperatur im Bereich von 50 bis 121°C unter Erhöhung des Partialdampfdruckes des Wassers, Methanols oder Äthanols behandelt werden.

Da die notwendigen Untersuchungen über Hepatitis-Virusinaktivierung in Blutprodukten nicht sofort beim Menschen vorgenommen werden können und Schimpansen nur in ungenügender Zahl zur Verfügung stehen, erfolgt erfindungsgemäß die Bewertung der Wirksamkeit der Inaktivierung mit Hilfe von Modellviren. Am Beispiel von Hundehepatitisvirus als Modellvirus konnte gezeigt werden, daß die eingangs erwähnten bekannten Verfahren zur Virusinaktivierung von Blutprodukten dem Inaktivierungsverfahren in Albumin weit unterlegen sind. So wird z. B. Hundehepatitisvirus in Proteinlösungen in Anwesenheit von 0,50 Saccharose und 2M Glycin als Stabilisatoren nicht inaktiviert; ebenso nicht in Faktor VIII-Präparationen in trockenem Zustand bei Erhitzen auf 60°C während 10 h. Hundehepatitisviren werden jedoch in Albuminlösungen bei Erhitzung auf 60°C während 10 h vollständig inaktiviert. Das gleiche gilt für E. Coli Bakteriophagen T4.

Bei der Bewertung des erfindungsgemäßen Verfahrens hat sich gezeigt, daß Hundehepatitisvirus und andere Modellviren vollständig inaktiviert werden. Die Inaktivierungsgeschwindigkeit von E. Coli Bakteriophagen T4 in beliebigen Blutprodukten in Anwesenheit der erfindungsgemäß wirksamen Menge an hydroxylgruppenhältigen Verbindungen ist sogar größer als in einer auf 60°C erhitzten Albuminlösung.

Vorzugsweise wird die Behandlung der Blutprodukte mit Wasserdampf bei einem Druck von 0,1 bis 2 bar durchgeführt.

Die vermehrungsfähigen filtrierbaren Krankheitserreger können u.a. Hepatitis-Viren oder Überträger von AIDS (acquired immune deficiency syndrome) sein.

Die Dauer der erfindungsgemäßen Hitzebehandlung kann in weiten Grenzen variiert werden, je nach der angewandten Temperatur und der Art bzw. Hitzeempfindlichkeit der zu behandelnden Blutprodukte. Sie kann zwischen 1 s und 100 h betragen.

Nach einer bevorzugten Ausführungsform wird die Behandlung der Blutprodukte bei einem Wassergehalt von 0,06 (6 Gew.-%) bis 0,30 (30 Gew.-%) bei einer Temperatur von 50 bis 90°C durchgeführt.

Das erfindungsgemäße Verfahren kann auch derart durchgeführt werden, daß die Behandlung der Blutprodukte in Gegenwart eines sauerstofffreien inerten Schutzgases, vorzugsweise Stickstoff, und allenfalls in Gegenwart von sauerstoffbindenden Substanzen durchgeführt wird.

Eine weitere bevorzugte Ausführungsform des erfindungsgemäßen Verfahrens besteht darin, daß die Blutprodukte in einem flüssigen Medium, in dem sie unlöslich sind, hitzebehandelt werden. Als flüssiges Medium kann Chloroform oder Essigsäureäthylester verwendet werden.

Das erfindungsgemäße Inaktivierungsverfahren läßt sich mit Vorteil bei einem Verfahren zur Herstellung von Blutprodukten, ausgewählt aus Enzymen, Proenzymen einschließlich Gerinnungsfaktoren, Enzyminhibitoren, Immunglobulinen, Albumin, Plasminogen, Fibrinogen, Fibronectin oder Plasma oder Mischungen einzelner Blutprodukte anwenden, wobei die Inaktivierung in einer beliebigen Stufe des Herstellungsverfahrens vorgenommen wird, worauf gegebenenfalls die Blutprodukte in eine galenische Zubereitung übergeführt werden.

In den Rahmen des erfindungsgemäßen Herstellungsverfahrens fällt auch die Arbeitsweise, daß ein Blutprodukt kovalent an eine wasserunlösliche Matrix gebunden und hitzeinaktiviert wird. Beispielsweise kann Immunglobulin an Sepharose immobilisiert und der Inaktivierung unterworfen werden. Anderseits ist es möglich, daß ein Blutprodukt auf einen gewebekompatiblen Träger, wie ein Kollagenvlies, nicht kovalent adsorbiert und hitzebehandelt wird. Eine besondere Ausführungsform bei der Herstellung einer fibrinogenhältigen Präparation besteht darin, daß auf einen gewebekompatiblen Träger, wie ein Kollagenvlies, eine fibrinogenhältige Komposition aufgebracht und hitzeinaktiviert wird.

Im Rahmen des erfindungsgemäßen Herstellungsverfahrens können auch weitere Fraktionierungsmaßnahmen angewendet werden, um die hitzeinaktivierten Blutprodukte von während der Hitzeinaktivierung allenfalls gebildeten Neoproteinen oder Neoantigenen zu befreien.

Weitere Merkmale des erfindungsgemäßen Inaktivierungsverfahrens sowie die Herstellung von Blutprodukten unter Anwendung des Verfahrens, die erreichten Wirkungen und die Überlegenheit gegenüber bekannten Verfahren ist in den folgenden Beispielen und Tabellen näher erläutert.

**Beispiel 1:**

a) Herstellung einer Faktor VIII-Präparation

6660 ml frisch gefrorenes Plasma wurde bei 0°C bis +4°C aufgetaut. Das entstandene Kryopräzipitat wurde durch Zentrifugieren abgetrennt und in 700 ml Trinatriumcitrat-Lösung, die 0,05 mg Natriumpentosansulfat pro ml und 30 Einheiten Aprotinin pro ml enthielt, gelöst. Der pH-Wert der Lösung wurde auf 6,3 und die Temperatur auf +4°C gestellt. Der entstandene Niederschlag wurde durch Zentrifugieren abgetrennt und verworfen.

Durch Zugabe von Äthanol bis zu einer Konzentration von wurde die Faktor VIII-hältige Fraktion ausgefällt. Der entstandene Niederschlag wurde durch Zentrifugieren abgetrennt und in einem Glycin-Citrat-NaCl-Puffer gelöst.

b) Inaktivierung eines Modellvirus

Die in beschriebener Weise erhaltene Faktor VIII-hältige Lösung wurde auf 50 mg Protein/ml eingestellt, mit einer Sindbis-Virussuspension in Zellkulturmedium TCM 199 bzw. mit virusfreiem TCM 199 versetzt und gefriergetrocknet. Das gefriergetrocknete Faktor VIII-Konzentrat wurde auf verschiedene Wassergehalte, u.zw. auf 0,08, auf 0,20, auf 0,30 und auf 0,50 eingestellt und in geschlossenen Behältern bei einer Temperatur von 60°C bzw. 80°C verschieden lang erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen.

Die Bestimmung des Virustiters wurde in folgender Weise durchgeführt:

Das Faktor VIII enthaltende Lyophilisat wurde in Wasser gelöst und mit isotoner Kochsalzlösung im Verhältnis 1 : 10 seriell verdünnt. Der Titer des Sindbis-Virus wurde durch Bewertung des zytopathischen Effektes auf sensitive Vero-Zellen in der Mikrotiterplatte bestimmt. Die Ergebnisse wurden nach statistischer Behandlung der Auswertung entsprechend der Formel von Reed und Muench als Logarithmus $TCID_{50}$ ausgedrückt (Reed J.L. and H. Muench; Amer.J.Hyg. 27, 493, (1938)).

c) Bestimmung der Restaktivität an virusfreien Proben

Die Bestimmung der Restaktivität an Faktor VIII erfolgte mit Hilfe des Thromboplastin-Bildungs-Tests (2-Stufen-Test). Die Faktor VIII-Restaktivität wurde berechnet durch Bildung des Quotienten aus der Faktor VIII-Aktivität der erhitzten Probe und der Faktor VIII-Aktivität der entsprechenden nicht erhitzten Probe.

d) Bestimmung des Wassergehaltes an virusfreien Proben

Das gefriergetrocknete Faktor VIII-Konzentrat wurde vor und nach der Hitzebehandlung mit wasserfreiem Methanol extrahiert. Der Wassergehalt der Methanollösung wurde nach Karl Fischer in einem Titrator nach dem coulometrischen Verfahren bestimmt (Scholz, E.; Fresenius' Z. anal. Chem. 314, 567 - 571, (1983)).

Die nach erfolgter Hitzebehandlung gemessenen Virustiter und die nach der Behandlung noch vorhandene Restaktivität sind der Tabelle I zu entnehmen, woraus sich deutlich ergibt, daß die Inaktivierungsgeschwindigkeit vom Wassergehalt abhängig ist. Während bei der Anwendung von einer Temperatur von 60°C die Nachweisgrenze des Virus bei einem Wassergehalt von 0,08 nach 3 h erreicht wird, wird diese Grenze bei einem Wassergehalt von 0,20 und 0,30 schon nach einer Stunde und bei einem Wassergehalt von 0,50 schon nach 0,3 h erreicht. Der Inaktivierungseffekt ist auch von der Temperatur abhängig. Bei einer Temperatur von 80°C und einem Wassergehalt von 0,08 wird die Nachweisgrenze des Virus schon nach 0,3 h erreicht.

Die Restaktivität nach der Inaktivierungsbehandlung war bei allen Proben mit einem Wassergehalt bis zu 0,30 mit mindestens 0,80 (80 %) zufriedenstellend, selbst bei einem Wassergehalt von 0,50 betrug die Restaktivität nach 3 h bei 60°C noch 0,38 (38 %).

Im Vergleich dazu wurden Faktor VIII-Konzentrate, die in gleicher Weise wie oben beschrieben hergestellt worden waren, in "trockenem Zustand" behandelt, d.h. mit einem nach dem Stand der Technik dem "trockenen Zustand" zugeordneten Wassergehalt von weniger als 0,05 Wasser. Die Ergebnisse sind in der Tabelle I a) angegeben, woraus zu ersehen ist, daß bei dem niedrigen Wassergehalt, der dem "trockenen Zustand" entspricht, die Nachweisgrenze des Virus bei einem Wassergehalt von 0,015 erst nach 10 h, bei einem Wassergehalt von 0,006 erst nach 30 h erreicht wird. Daraus ergibt sich, daß das erfindungsgemäße Verfahren 10- bis 100-fach wirksamer ist bei der Verminderung eines Virustiters um 3 bis 4 Zehnerpotenzen.

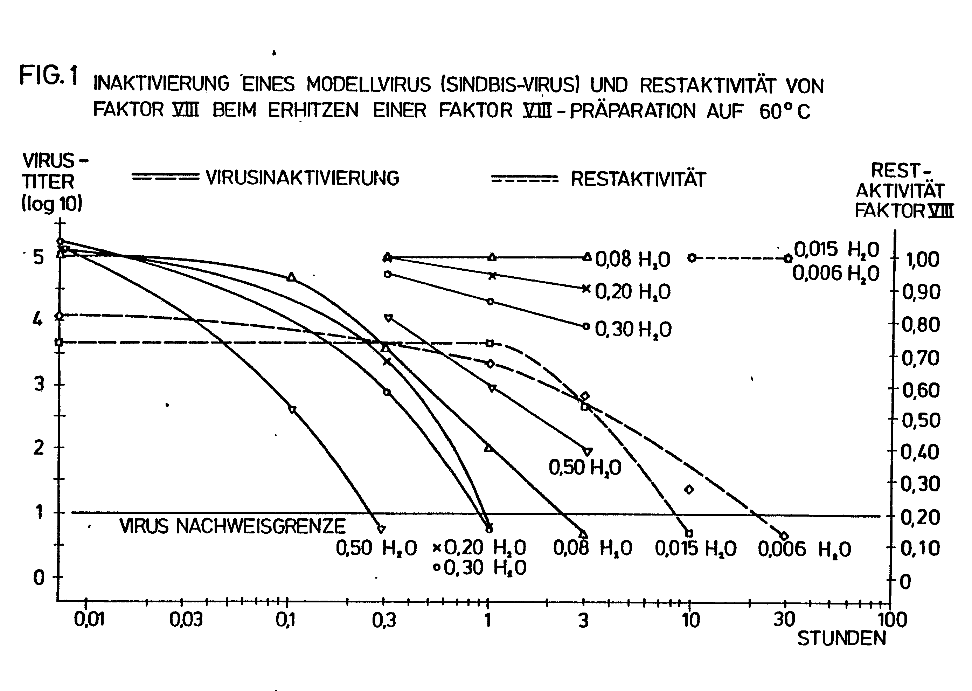

Diese Überlegenheit des erfindungsgemäßen Verfahrens gegenüber dem Stand der Technik ergibt sich anschaulich auch aus den Virusinaktivierungskurven in Fig. 1 der Zeichnung, wobei die voll ausgezogenen Kurven die erfindungsgemäße Inaktivierung bei 0,50, 0,30, 0,20 bzw. 0,08 $H_2O$ und die strichliert gezeichneten Kurven den Virustiterabfall in "trockenem Zustand" von 0,015 $H_2O$ und 0,006 $H_2O$ veranschaulichen. Auf der Abszisse der Fig. 1 ist die Zeitdauer der Behandlung in Stunden im logarithmischen Maßstab angegeben, auf der Ordinate der Virustiter im logarithmischen Maßstab. Zusätzlich ist in Fig. 1 der Verlauf der Restaktivität angegeben, wobei die voll ausgezogenen Kurven für den erfindungsgemäßen Wassergehalt und die strichlierten Kurven für den Restaktivitätsverlauf in trockenem Zustand stehen.

**Beispiel 2:**

Eine in gleicher Weise wie in Beispiel 1 hergestellte Faktor VIII-hältige Lösung wurde mit einer Sindbis-Virussuspension in Zellkulturmedium TCM 199 bzw. mit virusfreiem TCM 199 versetzt und gefriergetrocknet. Das gefriergetrocknete Faktor VIII-Konzentrat wurde in Gegenwart von Methanol bei einer Temperatur von 60°C der Inaktivierungsbehandlung unterworfen.

Die nach erfolgter Hitzebehandlung gemessenen Virustiter sind aus Tabelle II zu entnehmen, wobei in der Tabelle IIa die Vergleichsergebnisse ohne den erfindungsgemäßen Gehalt an Methanol angegeben sind. Wieder ist die Überlegenheit der erfindungsgemäßen Arbeitsweise deutlich, indem bei dieser die Inaktivierungsgeschwindigkeit des Modellvirus bei Erhaltung der Restaktivität wesentlich größer ist.

**Beispiel 3:**

a) Herstellung einer den partiellen Prothrombinkomplex enthaltenden Präparation:
Eine die Gerinnungsfaktoren II, IX und X enthaltende Präparation wurde nach der in Vox. Sang. 33, 37 - 50 (1977) beschriebenen Methode aus menschlichem Plasma durch Adsorption an DEAE-Sephadex, Waschen des Ionenaustauschers und Elution des Komplexes gewonnen.

b) Inaktivierung eines Modellvirus:
Das Eluat wurde dialysiert, gefriergetrocknet und daraus eine wässerige Lösung des partiellen Prothrombinkomplexes mit einem Gehalt von 50 mg Protein/ml bereitet. Die Lösung wurde mit einer Suspension des Bakteriophagen T4 in Kulturmedium für Escherichia coli 11303 bzw. mit virusfreiem Kulturmedium versetzt und gefriergetrocknet. Das gefriergetrocknete Konzentrat wurde auf einen Wassergehalt von 0,09 eingestellt. Geschlossene Behälter mit gefriergetrockneten partiellen Prothrombinkomplex-Proben - mit und ohne Virus - wurden bei einer Temperatur von 60°C verschieden lang erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen.

Die Bestimmung des Virustiters wurde in folgender Weise durchgeführt:
Das den partiellen Prothrombinkomplex enthaltende Lyophilisat wurde nach der Hitzebehandlung in Wasser gelöst und mit 1 mM $MgCl_2$ im Verhältnis 1 : 10 seriell verdünnt. Eine Mischung von 3 ml 0,7 % Bacto-Agar, 43 bis 45°C, 100 µl Suspension von E. coli in Flüssigmedium und 200 µl der bakteriophagenhältigen Probe bzw. Verdünnung wurden durchgemischt und rasch auf einer Nähragarplatte (ATCC 129) ausgegossen. Nach Inkubation über Nacht bei 37°C wurden mit Hilfe eines Zählgerätes die durch vermehrungsfähige Viruspartikel erzeugten Plaques im konfluenten Zellrasen gezählt (PFU, plaque-forming units). Die Ergebnisse wurden als $log_{10}$ PFU ausgedrückt.

c) Bestimmung der Restaktivität an virusfreien Proben
Die Bestimmung der Faktor IX-Aktivität erfolgte durch Zusatz der zu testenden Probe zu einem Faktor IX-Mangelplasma und Bestimmung der aktivierten partiellen Thromboplastinzeit (1-Stufen-Test). Die Faktor IX-Restaktivität einer erhitzten Probe wurde berechnet durch Bildung des Quotienten aus der Faktor IX-Aktivität der erhitzten Probe und der Faktor IX-Aktivität der entsprechenden nicht erhitzten Probe.

d) Bestimmung des Wassergehaltes an virusfreien Proben
Diese Bestimmung erfolgte in gleicher Weise, wie in Beispiel 1 beschrieben.

Die nach erfolgter Hitzebehandlung gemessenen Virustiter und die nach der Behandlung noch vorhandene Restaktivität sind der Tabelle III zu entnehmen, woraus sich ergibt, daß die Nachweisgrenze des Virus schon nach kurzer Zeit - weniger als 1 h - erreicht wurde, wobei die Restaktivität voll erhalten blieb. Im Vergleich dazu ist aus der Tabelle IIIa zu ersehen, daß bei einer Hitzebehandlung partieller Prothrombinkomplex-Präparationen in "trockenem Zustand" bei einem Wassergehalt von 0,004 selbst nach zehnstündiger Behandlung der Virustiter mit 2,0 noch relativ hoch war, sogar bei Anwendung von höheren Temperaturen von 80 und 90°C.

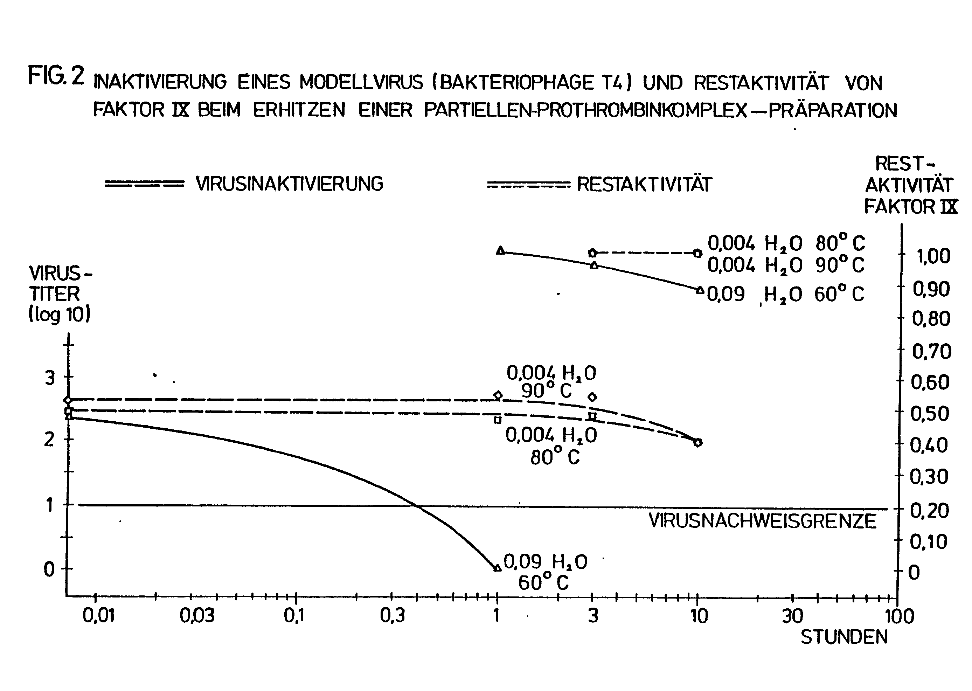

Die Virusinaktivierungskurven und der Verlauf der Restaktivitäten nach diesem Beispiel sind in Fig. 2 in gleicher Weise wie in Fig. 1 veranschaulicht mit voll ausgezogenen Linien dargestellt, wogegen die nach dem Stand der Technik im trockenen Zustand erhaltenen Kurven strichliert angegeben sind.

**Beispiel 4:**

a) Herstellung einer den totalen Prothrombinkomplex enthaltenden Präparation
Eine den Gerinnungsfaktor VII enthaltende Präparation wurde nach der in der AT-PS 359.646 beschriebenen Methode aus menschlichem Zitratplasma hergestellt. Nach Abtrennung des Kryopräzipitates und einer DEAE-Sephadexbehandlung wurde Faktor VII an $Al(OH)_3$ adsorbiert. $Al(OH)_3$ wurde einem Waschprozeß und einem Faktor VII-Elutionsprozeß bei erhöhter Ionenstärke unterworfen. Das Faktor VII-hältige Eluat wurde dialysiert und mit dem dialysierten partiellen Prothrombinkomplex-Präparat, hergestellt nach Beispiel 3, in solchem Verhältnis gemischt, daß die Gerinnungsaktivitäten der Faktoren II, IX, X und VII ungefähr gleich hoch waren.

b) Inaktivierung eines Modellvirus

Die in beschriebener Weise erhaltene Lösung des totalen Prothrombinkomplexes wurde auf 50 mg Protein/ml eingestellt und mit einer Sindbis-Virussuspension in Zellkulturmedium TCM 199 bzw. mit virusfreiem TCM 199 versetzt und gefriergetrocknet. Das gefriergetrocknete Konzentrat wurde auf einen Wassergehalt von 0,07 eingestellt. Geschlossene Behälter mit den gefriergetrockneten totalen Prothrombinkomplex-Proben - mit und ohne Virus - wurden bei einer Temperatur von 60°C verschieden lang erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen.

Die Bestimmung des Virustiters erfolgte in gleicher Weise, wie in Beispiel 1 beschrieben.

c) Bestimmung der Restaktivität an virusfreien Proben

Die Bestimmung der Faktor IX-Aktivität erfolgte durch Zusatz der zu testenden Probe zu einem Faktor IX-Mangelplasma und Bestimmung der aktivierten partiellen Thromboplastinzeit (1-Stufen-Test). Die Faktor IX-Restaktivität einer erhitzten Probe wurde berechnet durch Bildung des Quotienten aus der Faktor IX-Aktivität der erhitzten Probe und der Faktor IX-Aktivität der entsprechenden nicht erhitzten Probe.

d) Bestimmung des Wassergehaltes an virusfreien Proben

Diese Bestimmung erfolgte in gleicher Weise, wie in Beispiel 1 beschrieben.

Die nach erfolgter Hitzebehandlung gemessenen Virustiter und die nach der Behandlung noch vorhandene Restaktivität sind aus der Tabelle IV zu entnehmen, woraus sich ergibt, daß nach einer Behandlungszeit von 3 h die Nachweisgrenze des Virus erreicht wurde, wobei die Restaktivität im wesentlichen erhalten blieb.

**Beispiel 5:**

a) Herstellung einer FEIBA-Präparation

Eine FEIBA enthaltende Präparation wurde aus frisch gefrorenem menschlichem Zitratplasma nach der in der AT-PS 368.883 beschriebenen Methode hergestellt, indem nach Auftauen des Plasmas, Abtrennen des dabei anfallenden Kryopräzipitates und Generieren der FEIB-Aktivität die Gerinnungsfaktoren durch Adsorption an einen Ionenaustauscher und Elution gewonnen wurden.

b) Inaktivierung eines Modellvirus

Die erhaltene FEIBA-hältige Lösung wurde auf 50 mg Protein/ml eingestellt und mit einer Sindbis-Virussuspension in Zellkulturmedium TCM 199 bzw. mit virusfreiem TCM 199 versetzt und gefriergetrocknet. Das gefriergetrocknete FEIBA-Konzentrat wurde auf verschiedene Wassergehalte, u.zw. auf 0,07, 0,08 bzw. auf einen Äthanolgehalt von 0,10 eingestellt und in geschlossenen Behältern bei verschiedenen Temperaturen, nämlich 60°C und 90°C, verschieden lang erhitzt. Je drei Proben wurden vor dem Erhitzen und zu bestimmten Zeiten während des Erhitzungsvorganges zur Messung des Virustiters einerseits und zur Bestimmung der Restaktivität und des Wassergehaltes andererseits entnommen.

Die Bestimmung des Virustiters erfolgte in gleicher Weise, wie in Beispiel 1 beschrieben.

c) Bestimmung der Restaktivität an virusfreien Proben

Die Bestimmung der FEIB-Restaktivität erfolgte, wie in der AT-PS 350.726 beschrieben, durch Zusatz der zu testenden Probe zu einem Faktor VIII-Inhibitorplasma und Bestimmung der aktivierten partiellen Thromboplastinzeit (1-Stufen-Test). Die FEIB-Restaktivität einer erhitzten Probe wurde berechnet durch Bildung des Quotienten aus der FEIB-Aktivität der erhitzten Probe und der FEIB-Aktivität der entsprechenden nicht erhitzten Probe.

d) Bestimmung des Wassergehaltes an virusfreien Proben

Diese Bestimmung erfolgte in gleicher Weise, wie in Beispiel 1 beschrieben.

Die nach erfolgter Hitzebehandlung gemessenen Virustiter und die nach der Behandlung noch vorhandene Restaktivität sind der Tabelle V zu entnehmen, woraus sich ergibt, daß die Inaktivierungsgeschwindigkeit sowohl vom Wassergehalt als auch von der Inaktivierungstemperatur abhängig ist. Bei einem Wassergehalt von 0,07 und einer Temperatur von 60°C wird die Nachweisgrenze des Virus nach 3 h und bei einem Wassergehalt von 0,08 und einer Temperatur von 90°C wird die Nachweisgrenze schon nach 0,2 h erreicht. Die Restaktivität ist in allen Fällen zufriedenstellend.

Im Vergleich dazu ist in Tabelle Va ein Vergleichsbeispiel angegeben, wobei eine FEIBA-Probe in "trockenem Zustand" mit einem Wassergehalt von 0,009 bei 60°C behandelt worden ist. Der Virustiter ist selbst nach 30 h Behandlung noch mit 1,7 relativ hoch.

Die Inaktivierung einer nach diesem Beispiel hergestellten FEIBA-Präparation wurde zusätzlich an einem zweiten Modellvirus, nämlich an Hundehepatitis-Virus, geprüft, wobei das gefriergetrocknete FEIBA-Konzentrat auf einen Wassergehalt von 0,07 eingestellt wurde und die Behandlungstemperatur 60 bzw. 80 C betrug. Die nach erfolgter Hitzebehandlung gemessenen Virustiter sind der Tabelle VI zu entnehmen. Der Titer des Hundehepatitis-Virus wurde durch Bewertung des zytopathischen Effektes auf sensitive MDCK-Zellen in der Mikrotiterplatte bestimmt. Die Ergebnisse wurden nach statistischer Behandlung der Auswertung entsprechend der Formel von Reed und Muench als Logarithmus $TCID_{50}$ ausgedrückt (Reed J.L. and H. Muench; Amer.J.Hyg. 27, 493, (1938)). Aus der Virustiterbestimmung ergabt sich, daß bei beinem Wassergehalt von 0,07 und einer Temperatur von 60°C die Nachweisgrenze des Virus nach 1 h, bei einer Temperatur von 80° C schon nach 0,1 h erreicht wurde.

In der Tabelle VIa ist wieder ein Vergleichsbeispiel angegeben, wobei die Behandlung in "trockenem Zustand" erfolgte, mit dem Ergebnis, daß noch nach 30 h Behandlungszeit die Inaktivierung unzureichend war.

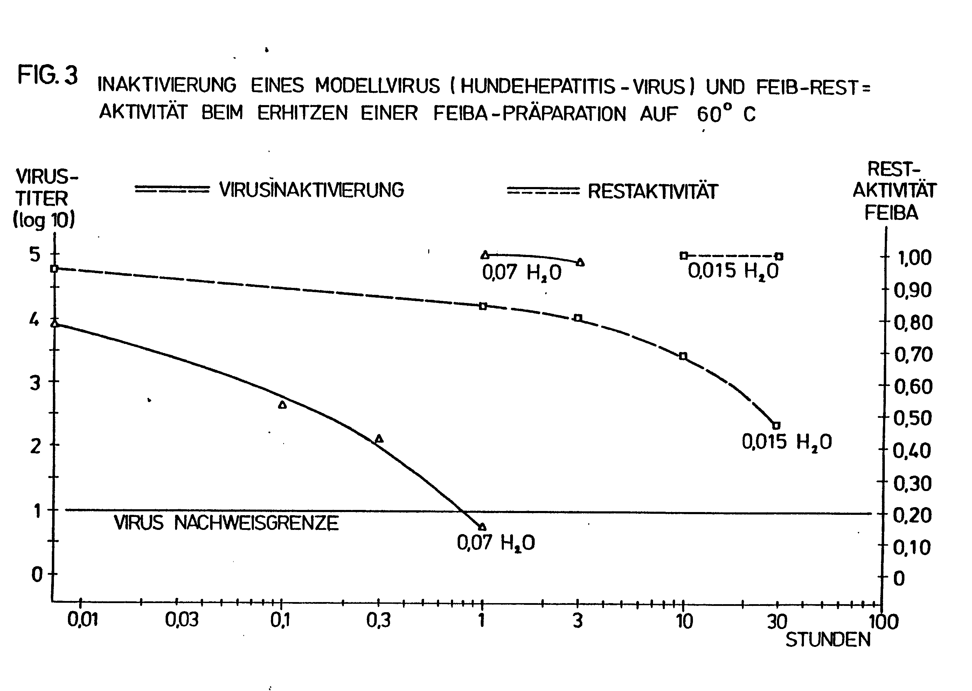

In Fig. 3 sind in gleicher Darstellung wie in Fig. 1 die Virusinaktivierungskurven und die Restaktivitäten nach diesem Beispiel veranschaulicht.

**Beispiel 6:**

a) Herstellung einer Immunglobulin-hältigen Präparation

Eine Immunglobulin-hältige Präparation wurde nach der von Oncley et al. im Journal of Amer.Chem.Soc., 71, 541 - 550 (1949) beschriebenen Alkohol-Fraktionierungsmethode aus menschlichem Plasma gewonnen. Die Lösung enthielt 100 mg Protein, 14 mg Glycin und 1,9 mg NaCl pro ml.

b) Inaktivierung eines Modellvirus

Die erhaltene wässerige Lösung wurde mit einer Sindbis-Virussuspension in Zellkulturmedium TCM 199 bzw. mit virusfreiem TCM 199 versetzt und gefriergetrocknet. Das gefriergetrocknete Immunglobulinkonzentrat wurde auf einen Wassergehalt von 0,054 eingestellt und in geschlossenen Behältern - mit und ohne Virus - bei einer Temperatur von 60°C verschieden lang erhitzt. Je drei Proben wurden vor dem Erhitzen und zu bestimmten Zeiten während des Erhitzungsvorganges zur Messung des Virustiters einerseits und zur Bestimmung der molekularen Integrität und des Wassergehaltes andererseits entnommen.

Die Bestimmung des Virustiters erfolgte in gleicher Weise, wie in Beispiel 1 beschrieben.

c) Bestimmung der molekularen Integrität an virusfreien Proben

Die Bestimmung erfolgte durch Vergleich der Proteinzusammensetzungen der erhitzten und der nicht erhitzten Probe, wobei die Trennung der Proteingemische im elektrischen Feld mittels $c_1$) Zelluloseacetat-Elektrophorese und $c_2$) SDS-Polyacrylamid-Gel-Elektrophorese (SDS-PAGE) erfolgte.

$c_1$) Mikrozonen-Elektrophorese auf Zelluloseacetat-Membran (Beckman-System)

Die Elektrophorese erfolgte nach dem Beckman-System (Microzone Electrophoresis Manual, Beckman Instructions 015-083630-C, 1977), wobei die Proben auf einer mit Veronalpuffer (pH 8,6) äquilibrierten Zelluloseacetat-Membran aufgetragen und hierauf die Proteine bei 250 V (Stromstärke 3 - 4 mA pro Membran, Laufzeit 20 min) getrennt wurden. Nach Fixierung erfolgte Färbung mit Ponceau S, Trocknung und densitometrische Auswertung der Membran.

$c_2$) SDS-Polyacrylamid-Gel-Elektrophorese (SDS-PAGE)

Die elektrophoretische Trennung der mit Sodium-dodecylsulfat (SDS) beladenen Proteine erfolgte in einem 5 % Polyacrylamid-Gel nach der von Weber und Osborn in "The Reliability of Molecular Weight Determinations by Dodecyl Sulfate-Polyacrylamide Gel Electrophoreses", J.Biol.Chem. 244, 4406 (1969) beschriebenen Methode. Die Färbung der getrennten Proteine erfolgte nach der von Merril et al. in "Ultrasensitive Stain for Proteins in Polyacrylamide Gels Shows Regional Variation in Cerebrospinal Fluid Proteins", Science 211, 1437 (1981) beschriebenen Methode mit Hilfe des Silberfärbungs-Reagentiensatzes der Firma BIO-RAD (Bulletin 1089).

d) Bestimmung des Wassergehaltes an virusfreien Proben

Diese Bestimmung erfolgte in gleicher Weise, wie in Beispiel 1 beschrieben.

Die nach erfolgter Hitzebehandlung gemessenen Virustiter und das Ergebnis der molekularen Integritätsbestimmung sind aus der Tabelle VII zu entnehmen.

**Beispiel 7:**

a) Herstellung einer $C_1$-Esterase-Inhibitor-Präparation

Eine $C_1$-Esterase-Inhibitor-hältige Präparation wurde aus humanem Plasma nach der in Vox. Sang. 26, 118 (1974) beschriebenen Methode durch Adsorption an einem Anionenaustauscher (DEAE-Sephadex) und anschließende Elution gewonnen. Nach Salzfällung zur Abtrennung unerwünschter Proteine wurde die gereinigte $C_1$-Esterase-Inhibitor-Präparation gefriergetrocknet.

b) Inaktivierung eines Modellvirus

Eine aus dem Lyophilisat der $C_1$-Esterase-Inhibitor-Präparation erhaltene wässerige Lösung wurde auf 50 mg Protein/ml eingestellt und mit einer Sindbis-Virussuspension in Zellkulturmedium TCM 199 bzw. mit virusfreiem TCM 199 versetzt und neuerlich gefriergetrocknet. Das Konzentrat wurde auf einen Wassergehalt von 0,10 eingestellt und in geschlossenen Behältern - mit und ohne Virus - bei einer Temperatur von 60°C erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen.

Die Bestimmung des Virustiters erfolgte in gleicher Weise, wie in Beispiel 1 beschrieben.

c) Bestimmung der Restaktivität an virusfreien Proben

Die Aktivität des $C_1$-Esterase-Inhibitors wurde über seine Fähigkeit, die Hydrolyse des chromogenen Substrates CI-1 (Pentapharm) durch $C_1$-Esterase zu hemmen, nach M. Kleindel, H. Lang, A. Philapitsch, G. Wöber, "Thrombosis and Haemostasis", 50, 244 (1983) bestimmt.

d) Bestimmung des Wassergehaltes an virusfreien Proben

Diese Bestimmung erfolgte in der Weise, wie in Beispiel 1 beschrieben.

Die nach erfolgter Hitzebehandlung gemessenen Virustiter und die nach der Behandlung noch vorhandene Restaktivität sind aus Tabelle VIII zu entnehmen.

**Beispiel 8:**

a) Herstellung einer Plasminogen-hältigen Präparation

Eine Plasminogen-hältige Präparation wurde nach der von D.G. Deutsch und E.T. Mertz in Science 170, 1095 (1970) beschriebenen Methode hergestellt. 50 ml Lysin-Sepharose wurden in einer Säule mit 0,1 M Phosphat, pH 7,4, äquilibriert. 340 ml Plasma wurden mit Wasser auf 640 ml verdünnt und die Säule wurde mit dieser Lösung beladen. Nach der Entfernung von Begleitproteinen durch Waschung mit einer 0,3 M Phosphatlösung (pH 7,4) wurde Plasminogen mit 0,2 M 6-Aminocapronsäure (pH 7,4) eluiert. Die Plasminogen-enthaltende Lösung wurde zur Entfernung von 6-Aminocapronsäure dialysiert und anschließend gefriergetrocknet.

b) Inaktivierung eines Modellvirus

Eine aus dem Lyophilisat hergestellte wässerige Lösung des Plasminogens wurde auf 50 mg Protein/ml eingestellt und mit einer Sindbis-Virussuspension in Zellkulturmedium TCM 199 bzw. mit virusfreiem TCM 199 versetzt und neuerlich gefriergetrocknet. Das Konzentrat wurde auf einen Wassergehalt von 0,08 eingestellt und in geschlossenen Behältern - mit und ohne Virus - bei einer Temperatur von 60°C erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen.

Die Bestimmung des Virustiters erfolgte in gleicher Weise, wie in Beispiel 1 beschrieben.

c) Bestimmung der Restaktivität an virusfreien Proben

Diese Bestimmung erfolgte in der Weise, daß eine Plasminogen-hältige Probe nach der Methode von K.C. Robbins, L. Summaria, Methods in Enzymology, 19, 184 - 186 (1970) mit Streptokinase zu Plasmin aktiviert und die freigesetzte Plasminaktivität durch Quantitierung der aus Kasein freigesetzten TCA-löslichen Spaltprodukte bestimmt wurde.

Die nach erfolgter Hitzebehandlung gemessenen Virustiter und die nach der Behandlung noch vorhandene Restaktivität sind der Tabelle IX zu entnehmen, woraus sich ergibt, daß die Nachweisgrenze des Virus bei zufriedenstellender Restaktivität nach 5 h erreicht wurde.

**Beispiel 9:**

a) Herstellung einer Plasmapräparation

Blut von gesunden Spendern wurde in einem Blutabnahmebeutel, worin Trinatriumcitrat-Lösung enthalten war, abgenommen. Nachdem die Mischung sanft durchmischt wurde, wurde sie zentrifugiert. Das überstehende Plasma wurde abdekantiert; 400 ml Plasma wurden mit 22 ml einer 15 % Glycinlösung, pH 3,6, gemischt.

b) Inaktivierung eines Modellvirus

Das mit Glycin gepufferte Plasma wurde mit einer Sindbis-Virussuspension in Zellkulturmedium TCM 199 bzw. mit virusfreiem TCM 199 versetzt, gefriergetrocknet und auf einen Wassergehalt von 0,08 eingestellt. Geschlossene Behälter mit den Trockenplasmaproben - mit und ohne Virus - wurden bei 60°C verschieden lang erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen. Die Bestimmung des Virustiters erfolgte in gleicher Weise, wie in Beispiel 1 beschrieben.

c) Bestimmung der Restaktivität an virusfreien Proben
Diese Bestimmung erfolgte dadurch, daß die aktivierte partielle Thromboplastinzeit (aPTT) des Plasmas ermittelt wurde, indem die Gerinnungszeit eines Gemisches von je 0,1 ml Plasma, Kaolin/Phospholipid-Suspension und 0,025 molarem Calciumchlorid gemessen wurde. Die Restaktivität einer erhitzten Probe wurde berechnet durch Bildung des Quotienten aus der aPTT der nicht erhitzten Probe und der aPTT der entsprechenden erhitzten Probe.

d) Bestimmung des Wassergehaltes an virusfreien Proben
Diese Bestimmung erfolgte in gleicher Weise, wie in Beispiel 1 beschrieben.
Die nach erfolgter Hitzebehandlung gemessenen Virustiter und die nach der Behandlung noch vorhandene Restaktivität sind der Tabelle X zu entnehmen, woraus sich ergibt, daß die Nachweisgrenze des Virus bei zufriedenstellender Erhaltung der Restaktivität schon nach 0,3 h erreicht wurde.

**Beispiel 10:**

a) Herstellung einer Fibrinogen- und Fibronectin-hältigen Präparation
Nach Abtrennung des Kryopräzipitates und der Prothrombinkomplex-Komponenten aus menschlichem Plasma wurde der anfallende Überstand auf 0°C gekühlt und unter Rühren so viel Äthanol zugesetzt, bis eine Endkonzentration von 0,08 (8 %) erreicht wurde. Die Temperatur wurde auf -2°C und der pH-Wert auf 7,0 gestellt. Die Suspension wurde zentrifugiert und der erhaltene pastenartige Niederschlag, der Fibrinogen und Fibronechtin enthielt, so lange mit einer Citrat-Glukose, pH 6,9 - gepufferten 6,5 %-igen Äthanollösung gewaschen, bis im Überstand nicht mehr als 0,5 mg Protein/ml nachweisbar waren. Das gereinigte Fibrinogen wurde eingefroren und gefriergetrocknet, um Äthanol zu entfernen.

b) Inaktivierung eines Modellvirus
Eine wässerige Lösung des Lyophilisates wurde auf 50 mg Protein/ml eingestellt und mit einer Sindbis-Virussuspension in Zellkulturmedium TCM 199 bzw. mit virusfreiem TCM 199 versetzt und neuerlich gefriergetrocknet. Das Konzentrat wurde auf einen Wassergehalt von 0,08 eingestellt und in geschlossenen Behältern - mit und ohne Virus - bei einer Temperatur von 60°C erhitzt. Je drei Proben wurden vor dem Erhitzen und zu bestimmten Zeiten während des Erhitzungsvorganges zur Bestimmung des Virustiters einerseits und der Restaktivität und des Wassergehaltes andererseits entnommen.
Die Bestimmung des Virustiters erfolgte in gleicher Weise, wie in Beispiel 1 beschrieben.

c) Bestimmung der Restaktivität an virusfreien Proben
Diese Bestimmung erfolgte

$c_1$) mit einem Wirksamkeits-Test (Thrombinzeit), wobei die Wirksamkeit einer Fibrinogen-Lösung nach der U-S-Pharmacopeia, 16. Revision (USP XVI, 1960), Seite 298, bestimmt wurde, indem eine Probe der Fibrinogenlösung mit Calciumchlorid und Thrombin gemischt und die Gerinnungszeit bestimmt wurde. Die Restaktivität wurde berechnet durch Bildung des Quotienten aus der Gerinnungszeit der nicht erhitzten Probe und der Gerinnungszeit der erhitzten Probe.
und

$c_2$) mit der Bestimmung des clottierbaren Proteins. Das clottierbare Protein einer Fibrinogenlösung wurde nach der USP XVI, Seite 298 bestimmt, indem (1) der Gesamtproteingehalt der Fibrinogenlösung bestimmt wurde (Stickstoffbestimmung nach Kjeldahl) und (2) der Proteingehalt des nach Thrombinzusatz clottierten Fibrins nach der gleichen Methode (Kjeldahl) bestimmt wurde. Der Quotient aus dem Protein-Wert des Fibrins (2) und dem Protein-Wert des Gesamtproteins (1) ergab das clottierbare Protein.
Die Restaktivität wurde berechnet durch Bildung des Quotienten aus dem clottierbaren Protein der erhitzten Probe und dem clottierbaren Protein der nicht erhitzten Probe.

d) Bestimmung des Wassergehaltes an virusfreien Proben
Die Bestimmung des Wassergehaltes erfolgte in gleicher Weise, wie in Beispiel 1 beschrieben.
Die nach erfolgter Hitzebehandlung gemessenen Virustiter und die nach der Behandlung noch vorhandene Restaktivität sind der Tabelle XI zu entnehmen, woraus sich ergibt, daß die Nachweisgrenze des Virus nach 5 h erreicht wurde, wobei die Restaktivitäten nach den angegebenen Methoden zufriedenstellend waren.

**Beispiel 11:**

a) Herstellung einer Albumin-hältigen Präparation

Zu 10 l menschlichem Blutplasma wurden bei einem pH-Wert von 7,0 und einer Temperatur von -2°C 0,08 Äthanol zugesetzt, wobei ein Niederschlag, der Fibrinogen enthält, ausfiel. Nach Abtrennen des Niederschlages wurde die Äthanolkonzentration auf 0,25 erhöht und die Temperatur auf -6°C gesenkt. Der ausfallende Niederschlag, der Immunglobulin enthielt, wurde abgetrennt und die Äthanolkonzentration des Überstandes, bei einem pH-Wert von 6,5 und einer Temperatur von -8°C, auf 0,40 erhöht, wobei eine weitere Fällung erfolgte. Der Niederschlag wurde abgetrennt und verworfen. Zur Ausfällung von Albumin wurde bei gleicher Temperatur der pH-Wert des Überstandes auf 5,4 gestellt. Der Niederschlag wurde durch Zentrifugieren abgetrennt und einem weiteren Reinigungsschritt unterworfen, indem er in Wasser gelöst und die Äthanolkonzentration bei einem pH-Wert von 4,8 und einer Temperatur von -2°C auf 0,10 gestellt wurde. Das ausgefallene Globulin wurde abgetrennt und verworfen. Die Äthanolkonzentration des Überstandes wurde auf 0,40 erhöht, die Temperatur auf -8°C gesenkt und der pH-Wert auf 5,1 gestellt. Der Niederschlag ist Albumin und wurde durch Zentrifugieren gesammelt und gefriergetrocknet.

b) Inaktivierung eines Modellvirus

Eine wässerige Lösung des Lyophilisates wurde auf 50 mg Protein/ml eingestellt und mit Hundehepatitisvirus in Zellkulturmedium TCM 199 bzw. mit virusfreiem TCM 199 versetzt und gefriergetrocknet. Das gefriergetrocknete Albuminkonzentrat wurde auf einen Wassergehalt von 0,07 eingestellt und in geschlossenen Behältern - mit und ohne Virus - bei 60°C verschieden lang erhitzt. Je drei Proben wurden vor dem Erhitzen und zu bestimmten Zeiten während des Erhitzungsvorganges zur Messung des Virustiters einerseits und der molekularen Integrität und des Wassergehaltes andererseits entnommen.

Die Bestimmung des Virustiters erfolgte in gleicher Weise, wie in Beispiel 5 beschrieben. Die Bestimmung der molekularen Integrität, nämlich des Verhaltens in der Zelluloseacetat-Elektrophorese und SDS-Polyacrylamid-Gel-Elektrophorese, erfolgte in gleicher Weise, wie in Beispiel 6 beschrieben.

c) Bestimmung des Wassergehaltes an virusfreien Proben

Diese Bestimmung erfolgte in gleicher Weise, wie in Beispiel 1 beschrieben.

Die nach erfolgter Hitzebehandlung gemessenen Virustiter und die molekulare Integrität sind aus der Tabelle XII zu entnehmen, woraus sich ergibt, daß die Nachweisgrenze des Virus schon nach einer Stunde erreicht wurde und die molekulare Integrität gegenüber nicht erhitzten Kontrollproben keine Veränderung zeigte.

**Beispiel 12:**

Herstellung von Blutpräparationen unter Verwendung eines flüssigen Mediums, in dem sie unlöslich sind

Eine wie in Beispiel 1a) hergestellte Faktor VIII-Präparation wurde auf 50 mg Protein/ml eingestellt, mit einer Sindbis-Virussuspension in Zellkulturmedium TCM 199 bzw. mit virusfreiem TCM 199 versetzt und gefriergetrocknet. Das gefriergetrocknete Faktor VIII-Präparat wurde auf einen Wassergehalt von 0,09 eingestellt. Mehrere Behälter mit den gefriergetrockneten Faktor VIII-Proben - mit und ohne Virus - wurden mit je 10 ml wassergesättigtem Chloroform (um den Wassergehalt der Proben nicht zu verändern) versetzt, verschlossen und 10 h lang bei 60°C erhitzt. Je drei Proben wurden vor dem Erhitzen und zu bestimmten Zeiten während des Erhitzungsvorganges zur Messung des Virustiters einerseits und der Restaktivität und des Wassergehaltes andererseits entnommen.

Vor der Bestimmung des Virustiters und der Restaktivität wurden die Proben vom Lösungsmittel durch Absaugen im Vakum befreit.

Die Bestimmung des Virustiters und des Wassergehaltes erfolgte wie in Beispiel 1 beschrieben.

Die nach erfolgter Hitzebehandlung gemessenen Virustiter und die nach der Behandlung vorhandene Restaktivität sind der Tabelle XIII zu entnehmen, woraus sich ergibt, daß der Virustiter nach 3 h unter die Nachweisgrenze absank, während die Restaktivität 0,85 betrug.

In gleicher Weise wurde die Inaktivierung von Hundehepatitisvirus in einer FEIBA- und einer partiellen Prothrombinkomplex-Präparation geprüft, wobei die FEIBA-Präparation nach Beispiel 5a) und die partielle Prothrombinkomplex-Präparation nach Beispiel 3a) hergestellt waren. Die Bestimmung des Virustiters erfolgte, wie in Beispiel 5 beschrieben. Die nach erfolgter Hitzebehandlung gemessenen Virustiter sind aus der Tabelle XIII zu entnehmen.

In den folgenden Beispielen wird die Herstellung von Blutprodukten beschrieben, wobei die erfindungsgemäße Inaktivierung angewendet wird, um etwa vorhandene vermehrungsfähige filtrierbare Krankheitserreger unschädlich zu machen.

**Beispiel 13:**

Herstellung einer Faktor VIII-Präparation

100 l frisch gefrorenes Plasma wurden bei 0°C bis +4°C aufgetaut. Das entstandene Kryopräzipitat wurde durch Zentrifugieren abgetrennt und in 10,5 l Trinatriumcitrat-Lösung, die 50 mg Natriumpentosansulfat/l und 30.000 Einheiten Aprotinin/l enthielt, gelöst. Der pH-Wert der Lösung wurde auf 6,3 und die Temperatur auf +4°C gestellt. Der entstandene Niederschlag wurde durch Zentrifugieren abgetrennt und verworfen.

Durch Zugabe von Äthanol bis zu einer Konzentration von 0,08 wurde die Faktor VIII-hältige Fraktion ausgefällt. Der entstandene Niederschlag wurde durch Zentrifugieren abgetrennt, in einem Glycin-Citrat-NaCl-Puffer gelöst, gefriergetrocknet, auf einen Wassergehalt von 0,08 gebracht und während 10 h bei 60°C erhitzt. Die Faktor VIII-Aktivitätsbestimmung nach dem Erhitzungsvorgang ergab eine Restaktivität von 0,92 (92 %) verglichen mit nicht erhitztem Material.

Zur Herstellung einer galenischen Zubereitung wurde das inaktivierte Pulver mit destilliertem Wasser unter Zusatz von Glycin-Trinatriumcitrat-NaCl so gelöst, daß eine Lösung mit 25 IE Faktor VIII pro ml, 10 g Glycin/l, 10 g Trinatriumcitrat · $2H_2O$/l und 5 g Natriumchlorid/l resultierte. Nach Einstellung des pH-Wertes auf 7,0 wurde die Lösung sterilfiltriert, mit 20 ml pro Flasche in die Endbehälter verfüllt und gefriergetrocknet. Die Faktor VIII-Aktivitätsbestimmung des gefriergetrockneten Materials in Endbehältern ergab 480 IE Faktor VIII/Flasche.

**Beispiel 14:**

Herstellung einer Faktor VIII-Präparation nach Entfernung von während der Inaktivierung gebildeten Neoproteinen

46 l frisch gefrorenes Plasma wurden bei 0°C bis +4°C aufgetaut. Das entstandene Kryopräzipitat wurde durch Zentrifugieren abgetrennt und in 960 ml einer Tri-Natriumcitratlösung bei 37°C gelöst. Der pH-Wert der Lösung wurde auf 6,3 und die Temperatur auf +4°C gestellt. Der entstandene Niederschlag wurde durch Zentrifugieren abgetrennt und verworfen. Der Faktor VIII-hältige Überstand wurde gefriergetrocknet, das Lyophilisat auf einen Wassergehalt von 0,075 (7,5 %) eingestellt und 10 h bei 60°C hitzeinaktiviert, um etwa vorhandene vermehrungsfähige filtrierbare Krankheitserreger, wie Hepatitisviren, zuverlässig unschädlich zu machen. Die Faktor VIII-Aktivitätsbestimmung nach dem Erhitzen ergab eine Restaktivität von 0,95 (95 %) Faktor VIII im Vergleich zu nicht erhitztem Material.

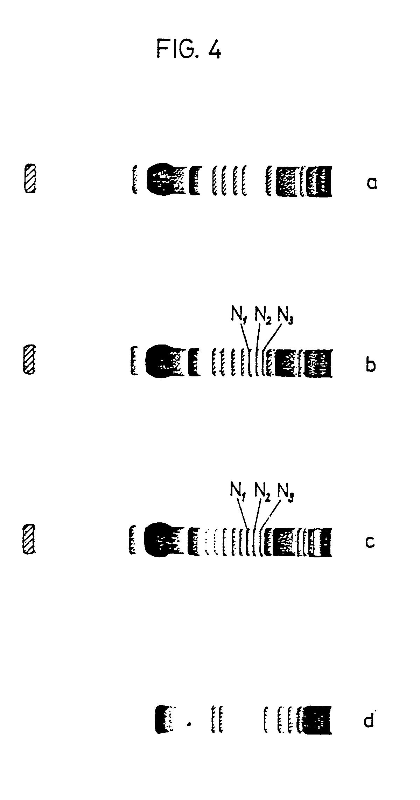

Proben der nicht erhitzten und der erhitzten gefriergetrockneten Faktor VIII-Präparation wurden mit Hilfe der SDS-Polyacrylamid-Gel-Elektrophorese untersucht. Bei der nicht erhitzten Probe wurde das in Fig. 4 mit a bezeichnete Bandenmuster erhalten. Bei der erhitzten Probe ergab sich das in Fig. 4 mit b bezeichnete Bandenmuster, bei welchem die mit $N_1$, $N_2$ und $N_3$ bezeichneten Banden in Erscheinung traten, die dem Auftreten von Neoproteinen während der Inaktivierungshitzebehandlung zuzuschreiben sind. Diese Neoproteine sind erfindungsgemäß durch eine Nachbehandlung der erhitzten Faktor VIII-Präparationen zu entfernen. Die Nachbehandlung bzw. weitere Fraktionierung erfolgte durch Auflösen der erhitzten Faktor VIII-Präparation in 900 ml Wasser und Zugabe von Äthylalkohol bis zu einer Konzentration von 0,10. Dabei wurde eine Faktor VIII-hältige Fraktion ausgefällt, durch Zentrifugieren abgetrennt und in einem Glycin-Citrat-NaCl-Puffer gelöst. Die Lösung wurde in eine galenische Zubereitung übergeführt, wie in Beispiel 13 beschrieben.

Die SDS-Polyacrylamid-Gel-Elektrophorese der gereinigten Fraktion ergab das in Fig. 4 mit d bezeichnete Bandenmuster: die Untersuchung des Überstandes der 0,10-Alkoholfällung ergab das mit c bezeichnete Bandenmuster. Daraus ist ersichtlich, daß die Neoproteine $N_1$, $N_2$ und $N_3$ im Überstand der Alkoholfällung verblieben, während die gereinigte Faktor VIII-Präparation diese Banden nicht mehr enthielt. Bei der beschriebenen Alkoholfraktionierung werden auch andere Proteine entfernt. Die Faktor VIII-Aktivität in der fraktionierten Präparation ist im wesentlichen, nämlich zu 0,70, erhalten geblieben.

Die erfindungsgemäße Hitzeinaktivierungsbehandlung kann bis zu 100 h ausgedehnt werden, ohne die Aktivitäten zu verlieren. Die Stabilität wichtiger Gerinnungsfaktoren beim Erhitzen bis zu 100 h bei bestimmten, im Rahmen der Erfindung einzuhaltenden Temperatur- und Wassergehalte ist aus Tabelle XIV zu ersehen.

**Beispiel 15:**

Herstellung einer Faktor VIII-Präparation unter Verwendung von Schutzgas

Eine in gleicher Weise wie in Beispiel 1 hergestellte Faktor VIII-hältige Lösung wurde mit einer Sindbis-Virussuspension in Zellkulturmedium TCM 199 bzw. mit virusfreiem TCM 199 versetzt und gefriergetrocknet.

Das gefriergetrocknete Faktor VIII-Konzentrat wurde auf einen Wassergehalt von 0,08 (8 Gew.-%) eingestellt, durch dreimaliges Evakuieren auf 100 mbar und nachfolgendes Begasen mit Luft, Stickstoff, Helium bzw. Argon in verschiedene "Atmosphären" gebracht und in geschlossenen Behältern bei einer Temperatur von 80°C verschieden lang erhitzt. Von jeder Variante wurden je drei Proben vor dem Erhitzen und zu bestimmten Zeiten während des Erhitzungsvorganges zur Messung des Virustiters einerseits und der Restaktivität an Faktor VIII und des Wassergehaltes andererseits entnommen.

11

Die Bestimmung des Virustiters, der Restaktivität an Faktor VIII und des Wassergehaltes erfolgte, wie in Beispiel 1 beschrieben; die Resultate sind der Tabelle XV zu entnehmen. Daraus ist ersichtlich, daß die Restaktivitäten bzw. Ausbeuten an Faktor VIII beim Erhitzen in einer Inertgasatmosphäre (Stickstoff, Helium, Argon) wesentlich höher sind als beim Erhitzen in Luft. Die Geschwindigkeit der Virusinaktivierung ist hingegen unabhängig von der Atmosphäre, in der erhitzt wurde.

**Beispiel 16:**

Herstellung einer den partiellen Prothrombinkomplex enthaltenden Präparation unter Verwendung von Schutzgas

Eine in gleicher Weise wie in Beispiel 3 hergestellte Präparation, welche die Gerinnungsfaktoren II, IX und X enthielt, wurde mit einer Sindbis-Virussuspension in Zellkulturmedium TCM 199 bzw. mit virusfreiem TCM 199 versetzt und gefriergetrocknet. Das gefriergetrocknete Konzentrat wurde auf einen Wassergehalt von 0,08 eingestellt, durch dreimaliges Evakuieren auf 100 mbar und nachfolgendes Begasen mit Luft, Stickstoff, Helium und Argon in verschiedene "Atmosphären" gebracht und in geschlossenen Behältern bei einer Temperatur von 90°C verschieden lang erhitzt. Von jeder Variante wurden je drei Proben vor dem Erhitzen und zu bestimmten Zeiten während des Erhitzungsvorganges zur Messung des Virustiters einerseits und der Restaktivität an Faktor IX und des Wassergehaltes andererseits entnommen.

Die Bestimmung des Virustiters und des Wassergehaltes erfolgte, wie in Beispiel 1 beschrieben, die Bestimmung der Restaktivität an Faktor IX erfolgte, wie in Beispiel 3 beschrieben.

Die Resultate sind der Tabelle XVI zu entnehmen. Daraus ist ersichtlich, daß die Restaktivitäten bzw. Ausbeuten an Faktor IX beim Erhitzen in einer Inertgasatmosphäre (Stickstoff, Helium, Argon) wesentlich höher sind als beim Erhitzen in Luft. Die Geschwindigkeit der Virusinaktivierung ist hingegen unabhängig von der Atmosphäre, in der erhitzt wurde.

**Tabelle I**
Faktor VIII-Präparation

| Wasser-gehalt | Temperatur °C | Sindbis-Virustiter ($\log_{10}$ TCID$_{50}$) nach Erhitzen während | | | | | | | Restaktivität Faktor VIII nach Erhitzen während | | | | |
|---|---|---|---|---|---|---|---|---|---|---|---|---|---|
| | | 0 | 0,1 | 0,3 | 1 | 3 | 10 | 30 h | 0,3 | 1 | 3 | 10 | 30 h |
| 0,50 | 60 | 5,1 | 2,6 | < 1 | < 1 | < 1 | | | 0,80 | 0,59 | 0,38 | | |
| 0,30 | 60 | 5,2 | | 2,9 | < 1 | < 1 | | | 0,95 | 0,86 | 0,80 | | |
| 0,20 | 60 | 5,1 | | 3,4 | < 1 | < 1 | | | 1,00 | 0,95 | 0,92 | | |
| 0,08 | 60 | 5,0 | 4,7 | 3,6 | 2,0 | < 1 | | | 1,00 | 1,00 | 1,00 | | |
| 0,08 | 80 | 4,9 | | < 1 | < 1 | < 1 | | | 0,97 | 0,89 | | | |

**Tabelle I a)**

| | | 0 | 0,1 | 0,3 | 1 | 3 | 10 | 30 h | 0,3 | 1 | 3 | 10 | 30 h |
|---|---|---|---|---|---|---|---|---|---|---|---|---|---|
| 0,015 | 60 | 3,7 | | | 3,7 | 2,7 | < 1 | < 1 | | | | 1,00 | 1,00 |
| 0,006 | 60 | 4,1 | | | 3,4 | 2,9 | 1,4 | < 1 | | | | 1,00 | 1,00 |

**Tabelle II**
Faktor VIII-Präparation

| Gehalt an Methanol | Wasser | Temperatur °C | Sindbis-Virustiter ($\log_{10}$ TCID$_{50}$) nach Erhitzen während | | | | | Restaktivität Faktor VIII nach Erhitzen während | | | |
|---|---|---|---|---|---|---|---|---|---|---|---|
| | | | 0 | 1 | 3 | 10 | 30 h | 1 | 3 | 10 | 30 h |
| 0,10 | 0,005 | 60 | 4,8 | 4,9 | 3,5 | < 1,5 | < 1,5 | 1,00 | 1,00 | 1,00 | 0,90 |

**Tabelle IIa**

| | Wasser | Temperatur °C | 0 | 1 | 3 | 10 | 30 h | 1 | 3 | 10 | 30 h |
|---|---|---|---|---|---|---|---|---|---|---|---|
| | 0,005 | 60 | 5,1 | 5,3 | 4,6 | 3,6 | 2,1 | 1 | 1 | 1 | 1 |

**Tabelle III**
Präparation mit partiellem Prothrombinkomplex

| Gehalt an Wasser | Temperatur °C | Bakteriophagen T4-Virustiter ($\log_{10}$ PFU) nach Erhitzen während | | | | Restaktivität Faktor IX nach Erhitzen während | | |
|---|---|---|---|---|---|---|---|---|
| | | 0 | 1 | 3 | 10 h | 1 | 3 | 10 h |
| 0,09 | 60 | 2,4 | 0 | 0 | 0 | 1,00 | 0,95 | 0,88 |

**Tabelle IIIa**

| 0,004 | 80 | 2,5 | 2,3 | 2,4 | 2,0 | | 1,00 | 1,00 |
|---|---|---|---|---|---|---|---|---|
| 0,004 | 90 | 2,6 | 2,7 | 2,7 | 2,0 | | 1,00 | 1,00 |

**Tabelle IV**
Präparation mit totalem Prothrombinkomplex

| Gehalt an Wasser | Temperatur °C | Sindbis-Virustiter ($\log_{10}$ $TCID_{50}$) nach Erhitzen während | | | | | Restaktivität Faktor IX nach Erhitzen während | |
|---|---|---|---|---|---|---|---|---|
| | | 0 | 0,1 | 0,3 | 1 | 3 h | 1 | 3 h |
| 0,07 | 60 | 6,1 | 5,2 | 4,8 | 3,3 | < 1 | 1,00 | 0,97 |

**Tabelle V**
FEIBA-Präparation

| Gehalt an Wasser | Äthanol | Temperatur °C | Sindbis-Virustiter ($\log_{10}$ $TCID_{50}$ nach Erhitzen während) | | | | | | | | | Restaktivität FEIBA nach Erhitzen während | | | | | |
|---|---|---|---|---|---|---|---|---|---|---|---|---|---|---|---|---|---|
| | | | 0 | 0,05 | 0,1 | 0,2 | 0,3 | 1 | 3 | 10 | 30 h | 0,1 | 0,3 | 1 | 3 | 10 | 30 h |
| 0,007 | 0,10 | 60 | 3,9 | | | | | 3,5 | 2,8 | < 1 | < 1 | | | 1,0 | 1,0 | 1,0 | 0,9 |
| 0,07 | | 60 | 4,1 | | | 3,8 | 3,1 | 2,1 | < 1 | | | | | 1,0 | 0,98 | | |
| 0,08 | | 90 | 4,7 | 2,9 | | < 1 | < 1 | < 1 | < 1 | | | 0,95 | 0,88 | | | | |

**Tabelle Va**

| Gehalt an Wasser | | Temperatur °C | Sindbis-Virustiter ($\log_{10}$ $TCID_{50}$ nach Erhitzen während) | | | | | | | | | Restaktivität FEIBA nach Erhitzen während | | | | | |
|---|---|---|---|---|---|---|---|---|---|---|---|---|---|---|---|---|---|
| 0,009 | | 60 | 5,2 | | | | | 4,8 | 4,8 | 4,6 | 1,7 | | | 1,0 | 1,0 | 1,0 | 1,0 |

**Tabelle VI**
FEIBA-Präparation

| Gehalt an Wasser | Temperatur °C | Hundehepatitis-Virustiter ($\log_{10}$ $TGID_{50}$) nach Erhitzen während | | | | | | | Restaktivität FEIBA nach Erhitzen während | | | |
|---|---|---|---|---|---|---|---|---|---|---|---|---|
| | | 0 | 0,1 | 0,3 | 1 | 3 | 10 | 30 h | 1 | 3 | 10 | 30 h |
| 0,07 | 60 | 3,9 | 2,6 | 2,1 | < 1 | < 1 | | | 1,00 | 0,98 | | |
| 0,07 | 80 | 3,5 | < 1 | < 1 | < 1 | < 1 | | | 0,87 | 0,75 | | |

**Tabelle VIa**

| Gehalt an Wasser | Temperatur °C | Hundehepatitis-Virustiter ($\log_{10}$ $TGID_{50}$) nach Erhitzen während | | | | | | | Restaktivität FEIBA nach Erhitzen während | | | |
|---|---|---|---|---|---|---|---|---|---|---|---|---|
| 0,015 | 60 | 4,8 | | | 4,2 | 4,0 | 3,4 | 2,3 | | | 1,00 | 1,00 |

**Tabell VII**
Immunglobulin-hältige Präparation

| Gehalt an Wasser | Temperatur °C | Sindbis-Virustiter ($\log_{10}$ $TCID_{50}$) nach Erhitzen während | | | | | molekulare Integrität |
|---|---|---|---|---|---|---|---|
| | | 0 | 1 | 2 | 4 | 10 h | |
| 0,054 | 60 | 6,4 | 5,5 | 4,4 | 3,0 | < 1 | keine Veränderung gegenüber nicht erhitzten Kontrollproben |

**Tabelle VIII**
$C_1$Esterase-Inhibitor-Präparation

| Gehalt an Wasser | Temperatur °C | Sindbis-Virustiter ($\log_{10}$ $TCID_{50}$) nach Erhitzen während | | | | | Restaktivität (chromogenes Substrat) nach Erhitzen während | | | |
|---|---|---|---|---|---|---|---|---|---|---|
| | | 0 | 1 | 2 | 4 | 10 h | 1 | 2 | 4 | 10 h |
| 0,10 | 60 | 6,4 | 5,0 | 4,5 | 3,0 | < 1 | 0,99 | 0,98 | 0,96 | 0,93 |

**Tabelle IX**

| Gehalt an Wasser | Temperatur °C | Sindbis-Virustiter ($log_{10}$ $TCID_{50}$) nach Erhitzen während | | | | Restaktivität Plasminogen nach Erhitzen während | | |
|---|---|---|---|---|---|---|---|---|
| | | 0 | 1 | 2 | 5 h | 1 | 2 | 5 h |
| 0,058 | 60 | 5,6 | 4,5 | 3,7 | < 1,5 | - | 0,94 | 1,02 |

**Tabelle X**

Plasmapräparation

| Gehalt an Wasser | Temperatur °C | Sindbis-Virustiter ($log_{10}$ $TCID_{50}$) nach Erhitzen während | | | Restaktivität aPTT nach Erhitzen während | |
|---|---|---|---|---|---|---|
| | | 0 | 0,3 | 1 h | 0,3 | 1 h |
| 0,052 | 60 | 5,4 | < 1 | < 1 | 0,97 | 0,91 |

**Tabelle XI**

Fibrinogen- und Fibronectinhältige Präparation

| Gehalt an Wasser | Temperatur °C | Sindbis-Virustiter ($log_{10}TCID_{50}$) nach Erhitzen während | | | | Restaktivität: Thrombinzeit ($c_1$) clottierbares Protein ($c_2$) nach Erhitzen während | | |
|---|---|---|---|---|---|---|---|---|
| | | 0 | 1 | 2 | 5 h | 1 | 2 | 5 h |
| 0,063 | 60 | 4,9 | 2,9 | 1,5 | < 1 | $c_1$) 0,80 $c_2$) 0,91 | 0,80 0,85 | 0,70 0,73 |

**Tabelle XII**

Albumin-hältige Präparation

| Gehalt an Wasser | Temperatur °C | Hundehepatitis-Virustiter ($log_{10}$ $TCID_{50}$) nach Erhitzen während | | | | molekulare Integrität |
|---|---|---|---|---|---|---|
| | | 0 | 1 | 2 | 4 h | |
| 0,07 | 60 | 3,2 | < 1 | < 1 | < 1 | keine Veränderung gegenüber nicht erhitzten Kontrollproben |

**Tabelle XIII**

Faktor VIII-Präparation

| Gehalt an Wasser | Temperatur °C | Sindbis-Virustiter ($log_{10}$ $TCID_{50}$) nach Erhitzen während | | | | Restaktivität Faktor VIII nach Erhitzen während 3 h |
|---|---|---|---|---|---|---|
| | | 0 | 0,3 | 1 | 3 h | |
| 0,09 | 60 | 3,9 | 4,6 | 2,9 | < 1 | 0,85 |

FEIBA-Präparation

| Gehalt an Wasser | Temperatur °C | Hundehepatitis-Virustiter ($log_{10}TCID_{50}$) nach Erhitzen während | | | | | Restaktivität FEIBA nach Erhitzen während 3 h |
|---|---|---|---|---|---|---|---|
| | | 0 | 0,1 | 0,3 | 1 | 3 h | |
| 0,08 | 60 | 2,9 | 2,0 | 2,0 | 2,1 | < 1 | 0,83 |

Partielle Prothrombinkomplex-Präparation

| Gehalt an Wasser | Temperatur °C | Hundehepatitis-Virustiter ($log_{10}TCID_{50}$) nach Erhitzen während | | | | | Restaktivität Faktor IX nach Erhitzen während 3 h |
|---|---|---|---|---|---|---|---|
| | | 0 | 0,1 | 0,3 | 1 | 3 h | |
| 0,08 | 60 | 2,6 | 1,8 | < 1 | < 1 | < 1 | 0,87 |

**Tabelle XIV**

Stabilität der Gerinnungsfaktoren

| Produkt | Gehalt an Wasser | Temperatur °C | Restaktivität nach Erhitzen während | | |
|---|---|---|---|---|---|
| | | | 10 | 30 | 100 h |
| Faktor VIII-Konzentrat | 0,08 | 60 | 0,94 | 0,85 | 0,47 Faktor VIII |
| FEIBA | 0,07 | 60 | 0,95 | 0,82 | 0,67 FEIBA |
| Totaler Prothrombinkomplex | 0,07 | 60 | 0,91 | 0,79 | 0,38 Faktor IX |
| Partieller Prothrombinkomplex | 0,09 | 60 | 0,88 | 0,73 | 0,33 Faktor IX |

**Tabelle XV**

Faktor VIII-Präparation

| Wassergehalt | Temperatur °C | Atmosphäre | Sindbis-Virustiter (logTCID$_{50}$) nach Erhitzen während | | | | | Restaktivität Faktor VIII nach Erhitzen während | | |
|---|---|---|---|---|---|---|---|---|---|---|
| | | | 0 | 0,3 | 1 | 3 | 10 h | 1 | 3 | 10 h |
| 0,08 | 80 | Luft | 4,8 | < 1 | < 1 | < 1 | < 1 | 0,85 | 0,53 | 0,28 |
| 0,08 | 80 | Stickstoff | 5,0 | < 1 | < 1 | < 1 | < 1 | 0,95 | 0,83 | 0,60 |
| 0,08 | 80 | Helium | 4,7 | < 1 | < 1 | < 1 | < 1 | 0,97 | 0,85 | 0,62 |
| 0,08 | 80 | Argon | 4,9 | < 1 | < 1 | < 1 | < 1 | 0,98 | 0,85 | 0,62 |

**Tabelle XVI**

Präparation mit partiellem Prothrombinkomplex

| Wassergehalt | Temperatur °C | Atmosphäre | Sindbis-Virustiter (TCID$_{50}$) nach Erhitzen während | | | | | Restaktivität Faktor IX nach Erhitzen während | | |
|---|---|---|---|---|---|---|---|---|---|---|
| | | | 0 | 0,3 | 1 | 3 | 10 h | 1 | 3 | 10 h |
| 0,08 | 90 | Luft | 4,7 | < 1 | < 1 | < 1 | < 1 | 0,63 | 0,30 | 0,07 |
| 0,08 | 90 | Stickstoff | 4,9 | < 1 | < 1 | < 1 | < 1 | 0,82 | 0,55 | 0,22 |
| 0,08 | 90 | Helium | 5,1 | < 1 | < 1 | < 1 | < 1 | 0,75 | 0,47 | 0,14 |
| 0,08 | 90 | Argon | 4,8 | < 1 | < 1 | < 1 | < 1 | 0,70 | 0,42 | 0,12 |

**Patentansprüche**

1. Verfahren zur Inaktivierung von vermehrungsfähigen filtrierbaren Krankheitserregern in Blutprodukten unter Anwendung erhöhter Temperatur, dadurch gekennzeichnet, daß die Blutprodukte in festem Zustand auf einen Gehalt an Wasser, Methanol oder Äthanol von mehr als 0,05 (5 Gew.-%) und weniger als 0,70 (70 Gew.-%), vorzugsweise weniger als 0,40 (40 Gew.-%), eingestellt und in einem geschlossenen Behälter bei einer Temperatur im Bereich von 50 bis 121°C unter Erhöhung des Partialdampfdruckes des Wassers, Methanols oder Äthanols behandelt werden.

2. Verfahren nach Anspruch 1, dadurch gekennzeichnet, daß die Behandlung der Blutprodukte mit Wasserdampf bei einem Druck von 0,1 bis 2 bar durchgeführt wird.

3. Verfahren nach den Ansprüchen 1 oder 2, dadurch gekennzeichnet, daß die vermehrungsfähigen filtrierbaren Krankheitserreger Hepatitis-Viren oder Überträger von AIDS (acquired immune deficiency syndrome) sind.

4. Verfahren nach einem der vorhergehenden Ansprüche, dadurch gekennzeichnet, daß die Blutprodukte während einer Dauer von 1 s bis 100 h behandelt werden.

5. Verfahren nach einem der vorhergehenden Ansprüche, dadurch gekennzeichnet, daß die Behandlung der Blutprodukte bei einem Wassergehalt von 0,06 (6 Gew.-%) bis 0,30 (30 Gew.-%) bei einer Temperatur von 50 bis 90°C durchgeführt wird.

6. Verfahren nach einem der vorhergehenden Ansprüche, dadurch gekennzeichnet, daß die Behandlung der Blutprodukte in Gegenwart eines sauerstofffreien inerten Schutzgases, vorzugsweise Stickstoff, und allenfalls in Gegenwart von sauerstoffbindenden Substanzen durchgeführt wird.

7. Verfahren nach einem der vorhergehenden Ansprüche, dadurch gekennzeichnet, daß die Blutprodukte mit Wasserdampf oder gasförmigem Methanol bzw. Äthanol behandelt werden.

8. Verfahren nach einem der vorhergehenden Ansprüche, dadurch gekennzeichnet, daß die Blutprodukte in einem flüssigen Medium, in dem sie unlöslich sind, behandelt werden.

9. Verfahren nach Anspruch 8, dadurch gekennzeichnet, daß als flüssiges Medium Chloroform oder Essigsäureäthylester verwendet wird.

10. Verfahren nach einem der vorhergehenden Ansprüche, dadurch gekennzeichnet, daß ein Blutprodukt verwendet wird, welches an eine Matrix kovalent oder nicht-kovalent gebunden ist.

11. Verfahren nach Anspruch 10, dadurch gekennzeichnet, daß ein Blutprodukt verwendet wird, welches auf einen gewebekompatiblen Träger, wie ein Kollagenvlies, aufgebracht ist.

12. Verfahren nach Anspruch 11, dadurch gekennzeichnet, daß eine fibrinogenhältige Komposition verwendet wird, welche auf einen gewebekompatiblen Träger, wie ein Kollagenvlies, aufgebracht ist.

13. Verfahren nach Anspruch 1, dadurch gekennzeichnet, daß die inaktivierten Blutprodukte durch weitere Fraktionierungsmaßnahmen von während der Inaktivierung allenfalls gebildeten Neoproteinen oder Neoantigenen befreit werden.

14. Verfahren zur Herstellung von Blutprodukten, ausgewählt aus Enzymen, Proenzymen, einschließlich Gerinnungsfaktoren, Enzyminhibitoren, Immunglobulinen, Albumin, Plasminogen, Fibrinogen, Fibronectin oder Plasma oder Mischungen einzelner Blutprodukte, unter Anwendung des Verfahrens nach den Ansprüchen 1 bis 13, dadurch gekennzeichnet, daß die Inaktivierung in einer beliebigen Stufe des Herstellungsverfahrens vorgenommen wird, worauf gegebenenfalls die Blutprodukte in eine galenische Zubereitung übergeführt werden.

### Claims

1. Method of inactivating reproducible filterable pathogens in blood products under application of an elevated temperature, characterised in that the blood products, in solid state, are adjusted to a content of water, methanol or ethanol of more than 0.05 (5 % by weight) and less than 0.70 (70 % by weight), preferably less than 0.40 (40 % by weight), and are treated in a closed container at a temperature in the range of from 50 to 121°C while increasing the partial vapour pressure of water, methanol or ethanol.

2. Method according to claim 1, characterised in that the treatment of the blood products is carried out with water vapour at a pressure of from 0.1 to 2 bar.

3. Method according to claims 1 or 2, characterised in that the reproducible filterable pathogens are hepatitis viruses or agents capable of transmitting AIDS (acquired immune deficiency syndrome).

4. Method according to one of the preceding claims, characterised in that the blood products are treated for a period of from 1 s to 100 h.

5. Method according to one of the preceding claims, characterised in that the treatment of the blood products is carried out at a water content of from 0.06 (6 % by weight) to 0.30 (30 % by weight) at a temperature of from 50 to 90°C.

6. Method according to one of the preceding claims, characterised in that the treatment of the blood products is carried out in the presence of an inert protective gas free of oxygen, preferably nitrogen, and optionally in the presence of oxygen-binding substances.

7. Method according to one of the preceding claims, characterised in that the blood products are treated with water vapour or gaseous methanol and ethanol, respectively.

8. Method according to one of the preceding claims, characterised in that the blood products are treated in a liquid medium in which they are insoluble.

9. Method according to claim 8, characterised in that as the liquid medium chloroform or acetic ethyl ester is used.

10. Method according to one of the preceding claims, characterised in that a blood product is used which is covalently or non-covalently bound to a matrix.

11. Method according to claim 10, characterised in that a blood product is used which is applied to a tissue-compatible carrier, such as a collagen fleece.

12. Method according to claim 11, characterised in that a fibrinogen-containing composition is used which is applied to a tissue-compatible carrier, such as a collagen fleece.

13. Method according to claim 1, characterised in that the inactivated blood products are freed from neoproteins or neoantigens possibly formed during inactivation by further fractionation measures.

14. Method of producing blood products, selected from the group consisting of enzymes, proenzymes, including coagulation factors, enzyme inhibitors, immunoglobulins, albumin, plasminogen, fibrinogen, fibronectin or plasma or mixtures of individual blood products, by applying the method of claims 1 to 13, characterised in that the inactivation is carried out at any desired stage of the production method, whereupon the blood products are, if desired, converted into a galenic preparation.

# EP 0 159 311 B1

## Revendications

1. Procédé pour l'inactivation d'agents pathogènes filtrables, susceptibles de se propager, dans des dérivés du sang avec application d'une température élevée, caractérisé en ce que les dérivés du sang sont ajustés à l'état solide à une teneur en eau, en méthanol ou en éthanol de plus de 0,05 (5 % en poids) et moins de 0,70 (70 % en poids, de préférence moins de 0,40 (40 % en poids), et traités dans un récipient fermé à une température dans la gamme de 50 à 121°C avec élévation de la pression partielle de vapeur de l'eau, du méthanol ou de l'éthanol.

2. Procédé selon la revendication 1, caractérisé en ce que le traitement des dérivés du sang est mis en oeuvre avec la vapeur d'eau sous une pression de 0,1 à 2 bars.

3. Procédé selon la revendication 1 ou 2, caractérisé en ce que les agents pathogènes filtrables, susceptibles de se propager, sont des virus d'hépatite ou des vecteurs du SIDA (syndrome d'immunodéficience acquise).

4. Procédé selon l'une quelconque des revendications précédentes, caractérisé en ce que les dérivés du sang sont traités pendant une durée de 1 s à 100 h.

5. Procédé selon l'une quelconque des revendications précédentes, caractérisé en ce que le traitement des dérivés du sang est mis en oeuvre à une teneur en eau de 0,06 (6 % en poids) à 0,30 (30 % en poids) à une température de 50 à 90°C.

6. Procédé selon l'une quelconque des revendications précédentes, caractérisé en ce que le traitement des dérivés du sang est mis en oeuvre en présence d'un gaz protecteur inerte exempt d'oxygène, de préférence l'azote, et au besoin en présence de substances fixant l'oxygène.

7. Procédé selon l'une quelconque des revendications précédentes, caractérisé en ce que les dérivés du sang sont traités par la vapeur d'eau ou le méthanol ou l'éthanol gazeux.

8. Procédé selon l'une quelconque des revendications précédentes, caractérisé en ce que les dérivés du sang sont traités dans un milieu liquide dans lequel ils sont insolubles.

9. Procédé selon la revendication 8 caractérisé en ce que l'on utilise comme milieu liquide le chloroforme ou l'acétate d'éthyle.

10. Procédé selon l'une quelconque des revendications précédentes, caractérisé en ce que l'on utilise un dérivé du sang qui est fixé par liaison covalente ou non covalente sur une matrice.

11. Procédé selon la revendication 10, caractérisé en ce que l'on utilise un dérivé du sang qui est appliqué sur un support compatible avec les tissus, tel qu'une nappe de collagène.

12. Procédé selon la revendication 11, caractérisé en ce que l'on utilise une composition contenant du fibrinogène, qui est appliquée sur un support compatible avec les tissus, tel qu'une nappe de collagène.

13. Procédé selon la revendication 1, caractérisé en ce que les dérivés du sang inactivés sont débarrassés par des mesures de fractionnement supplémentaires des néoprotéines ou néoantigènes formés à la rigueur pendant l'inactivation.

14. Procédé pour la fabrication de dérivés du sang, choisis parmi les enzymes, les proenzymes, y compris les facteurs de coagulation, les inhibiteurs d'enzymes, les immunoglobulines, l'albumine, le plasminogène, le fibrinogène, la fibronectine ou le plasma ou des mélanges de dérivés du sang individuels, avec application du procédé selon les revendications 1 à 13, caractérisé en ce que l'inactivation est effectuée dans un stade quelconque du procédé de fabrication, après quoi les dérivés du sang sont éventuellement transformés en une préparation galénique.

FIG.1 INAKTIVIERUNG EINES MODELLVIRUS (SINDBIS-VIRUS) UND RESTAKTIVITÄT VON FAKTOR VIII BEIM ERHITZEN EINER FAKTOR VIII - PRÄPARATION AUF 60°C

**FIG. 2** INAKTIVIERUNG EINES MODELLVIRUS (BAKTERIOPHAGE T4) UND RESTAKTIVITÄT VON FAKTOR IX BEIM ERHITZEN EINER PARTIELLEN-PROTHROMBINKOMPLEX—PRÄPARATION

══════ VIRUSINAKTIVIERUNG          ═ ═ ═ ═ RESTAKTIVITÄT

REST-AKTIVITÄT FAKTOR IX

VIRUS-TITER (log 10)

0,004 $H_2O$ 80° C
0,004 $H_2O$ 90° C
0,09 $H_2O$ 60° C

0,004 $H_2O$ 90° C

0,004 $H_2O$ 80° C

VIRUSNACHWEISGRENZE

0,09 $H_2O$ 60° C

0,01   0,03   0,1   0,3   1   3   10   30   100

STUNDEN

EP 0 159 311 B1

**FIG. 3** INAKTIVIERUNG EINES MODELLVIRUS (HUNDEHEPATITIS - VIRUS) UND FEIB-REST= AKTIVITÄT BEIM ERHITZEN EINER FEIBA-PRÄPARATION AUF 60° C

VIRUS-TITER (log 10) — VIRUSINAKTIVIERUNG ===== RESTAKTIVITÄT

REST-AKTIVITÄT FEIBA

0,07 $H_2O$ 0,015 $H_2O$

0,015 $H_2O$

VIRUS NACHWEISGRENZE

0,07 $H_2O$

STUNDEN

# FIG. 4